# FASCICULE DE BREVET EUROPEEN

(11) **EP 1 196 118 B1**
(45) Date de publication et mention de la délivrance du brevet: **06.10.2004**
(21) Numéro de dépôt: 00958608.2
(22) Date de dépôt: 25.07.2000
(51) Int. Cl.: A61F 2/38

(54) **NOUVELLE PROTHESE DU GENOU**
NEUARTIGE KNIEPROTHESE
NOVEL KNEE PROSTHESIS

(30) Priorité: 26.07.1999 FR 9909664
(43) Date de publication de la demande: 17.04.2002
(73) Titulaire: Bercovy, Michel, 75015 Paris (FR)
(72) Inventeur: Bercovy, Michel, 75015 Paris (FR)
(74) Mandataire: Jolly, Jean-Pierre
(86) Numéro de dépôt international: PCT/FR2000/002133
(87) Numéro de publication internationale: WO 2001/006961

(56) Documents cités:
- WO-A-98/46171
- FR-A- 2 621 243
- US-A- 5 609 639
- The Journal of Arthroplasty Vol.11 No.2 1996 pages 166 - 172......... A Correlative Study of the Geometry and Anatomy of the Distal Femur...S.G.Elias...February , 27 , 1990

## Description

La présente invention concerne une nouvelle prothèse de l'articulation du genou. Cette prothèse a plus particulièrement pour objet de comporter un dispositif comprenant des moyens de contact optimal, de stabilisation et de guidage entre les pièces mobiles au cours des mouvements tridirectionnels.

On rappelle qu'une prothèse du genou est un implant destiné à remplacer chirurgicalement une articulation de genou détruite.

Une prothèse de genou comporte des éléments solidaires de l'os, généralement métalliques, et des éléments de glissement, généralement en une matière plastique telle que le polyéthylène. Les éléments métalliques sont :
- une pièce fémorale, reproduisant à peu près la forme de l'articulation fémorale du genou ; elle est fixée à l'os, soit directement par une surface réhabitable, soit indirectement par le biais d'une résine acrylique ;
- une surface tibiale fixée à l'extrémité supérieure du tibia, soit directement, soit par l'intermédiaire d'une résine acrylique ; et
- une surface rotulienne fixée à la face postérieure de l'os rotulien, soit directement, soit par l'intermédiaire d'une résine acrylique.

Ces pièces métalliques sont généralement en chrome-cobalt, en titane ou en acier inoxydable ou en d'autres métaux et alliages notamment des céramiques.

Les pièces de glissement et d'amortissement séparant les pièces métalliques sont généralement en polyéthylène ; elles peuvent être soit rendues solidaires de la pièce tibiale ou plateau fixe, soit être mobiles par rapport à cette dernière avec un degré variable de mobilité en rotation (plan horizontal), en déplacement antéro-postérieur (plan sagittal), et en déplacement médio-latéral (plan frontal) ; qu'elles soient fixes ou mobiles, les pièces de glissement en polyéthylène dites "inserts" s'articulent avec la pièce fémorale par deux condyles fémoraux, l'interne et l'externe, ceux-ci ayant une certaine congruence avec les deux surfaces concaves respectives, dites cavités glénoïdes de l'insert en polyéthylène. Un prothèse est dite "à plateau" mobile lorsque l'insert a une rotation axiale libre. La prothèse conforme à l'invention est préférentiellement de ce type.

La rotation axiale libre est un corollaire de la congruence ; la congruence est un avantage tribologique majeur, grâce auquel les pressions et l'usure du polyéthylène constitutif de l'insert sont faibles, ce qui confère une durée de vie plus longue à la prothèse.

Dans des formes plus récentes de prothèse, l'appui s'effectue par trois surfaces, une troisième zone d'appui située entre les deux condyles étant en contact avec une saillie médiane située à la surface supérieure de l'insert. L'emboîtement de ces deux parties peut impliquer :
- soit un troisième condyle fémoral dans une troisième cavité à la face supérieure de l'insert ;
- soit un pion à la face supérieure de l'insert dans une cavité (boîtier) situé entre les deux condyles fémoraux ;
- soit différentes formes de cames situées entre les condyles fémoraux et prenant appui sur une éminence à la partie supérieure de l'insert.

Dans tous les cas, cependant, il existe des angulations dans un plan de coupe de face, rendant le contact entre pièces discontinu.

La partie antérieure de la prothèse fémorale (trochlée) s'articule par l'intermédiaire d'un médaillon en polyéthylène avec la rotule. Ce médaillon en polyéthylène est fixé à l'embase rotulienne ou bien il peut être mobile par rapport à cette dernière. Il existe une certaine congruence entre l'articulation du fémur et l'articulation de la rotule.

Une prothèse de genou ne doit pas reproduire les formes exactes d'une articulation anatomique. En effet, dans cette dernière, les ménisques ajustent la congruence entre les pièces fémorales et tibiale. Les ligaments croisés et latéraux coaptent ces pièces et assurent, en coopération avec la forme asymétrique des surfaces articulaires, un déplacement relatif dans les trois plans de l'espace (frontal, sagittal et horizontal ). Ces différents éléments anatomiques étant absents dans le genou prothétique, ce dernier doit répondre à trois types d'impératifs : les impératifs physiologiques, les impératifs tribologiques et les impératifs de stabilité.

### (1) Impératifs physiologiques

Les impératifs physiologiques du genou doivent être respectés et, en particulier, les déplacements et les positionnements respectifs du fémur par rapport au tibia : roulement-glissement avec recul du point de contact du fémur sur l'insert lors de la flexion (90°) et avancée du fémur lors de l'extension ( 0° ), ce qui conditionne le moment d'action du tendon rotulien, qui commande l'efficacité de la propulsion musculaire et par ce biais le confort du patient, la qualité de la marche, de la montée et de la descente des escaliers, du relèvement à partir d'une position basse. Ces rapports doivent être assurés au cours des mouvements, par la forme des surfaces articulaires des pièces prothétiques (déterminées par le dessin de la prothèse) et en particulier par un effet de came entre le fémur et l'insert, en coopération avec une tension équilibrée des ligaments latéraux de l'articulation du genou, déterminée par l'action du chirurgien et par une instrumentation ancillaire respectant cet impératif.

### (2) Impératifs tribologiques

Les impératifs tribologiques doivent être respectés afin que le contact entre les pièces métalliques fémorales et les inserts en polyéthylène tibiaux et rotuliens ne soient par générateurs de phénomènes d'usure du polyéthylène. En particulier, les contacts doivent être les plus congruents possibles, car les contacts ponctuels, linéaires, ou faiblement surfaciques, sont générateurs de pression élevée dans le polyéthylène et donc d'usure et de fluage de ce dernier.

Dans certaines formes de prothèses, l'insert en polyéthylène est fixe par rapport au tibia. Dans ce cas, les mouvements tangentiels de roulement-glissement, de cisaillement et tous les mouvements de rotation se passent dans la zone de contact entre les condyles fémoraux et les glènes tibiales. Lorsque ces contacts sont faiblement surfaciques, des pressions élevées peuvent être atteintes au niveau du polyéthylène. Les recommandations des constructeurs conseillent généralement de respecter une pression inférieure ou égale à 10MPa dans les zones de polyéthylène où l'appui est constant, c'est-à-dire dans les zones proches de la position de marche. Une pression idéale de 4 MPa est souhaitée. Or, dans les prothèses avec une congruence faible(surfaces articulaires linéaires ou ponctuelles entre fémur et insert en polyéthylène), des pressions supérieures à 30MPa sont couramment mesurées, pouvant même atteindre 50 MPa. Ceci concourt à une dégradation rapide du polyéthylène, pouvant nécessiter des réinterventions pour changement des implants prothétiques. Une surface de contact supérieure à 400mm² est conseillée. L'état de l'art, dans le domaine des prothèses articulaires, a conduit à fabriquer des prothèses dont les inserts en polyéthylène sont congruents par rapport à la surface fémorale, cette congruence ayant comme corollaire que l'insert doit être mobile en rotation par rapport à l'embase tibiale. Ces implants sont appelés prothèses à plateau mobile. Dans de tels cas, une congruence articulaire peut être respectée, permettant d'obtenir des contacts surfaciques élevés. Avec ce type de prothèse, on obtient couramment des pressions de l'ordre de 4 à 8 MPa dans le polyéthylène, ce qui favorise la durée de vie de ce dernier.

Différentes prothèses congruentes existent mais elles présentent toutes deux inconvénients majeurs, que la présente invention cherche à corriger :
- la congruence du contact entre les condyles et les glènes tibiales existe surtout près de l'extension, mais diminue au cours de la flexion ;
- la congruence n'est envisagée que dans un seul plan : le plan sagittal ; ainsi, des contraintes élevées peuvent survenir dans les zones de contact lors des mouvements en inclinaison ou roulis, en rotation, ou en combinaison dans ces trois directions, notamment lorsque les surfaces comportent des angulations.
Pour remédier à cela, les différentes surfaces doivent être tangentes les unes aux autres dans les deux plans de l'espace (sagittal et frontal), afin de permettre des glissements sans arrêts brutaux et sans contacts anguleux dans ces trois directions.

### (3) Les impératifs de stabilité

Le respect de la stabilité du genou porteur d'une prothèse peut être obtenu par différents mécanismes :
- conservation des deux ligaments croisés ;
- conservation du seul ligament croisé postérieur, mais il a été démontré que, dans ce cas, le déplacement antéro-postérieur ne respecte pas une cinématique satisfaisante ;
- enfin, stabilisation de la prothèse par un mécanisme central destiné à guider le fémur par rapport au tibia.

Ce dernier type de mécanisme est actuellement le plus efficace pour conserver la stabilité en accord avec la cinématique du genou et c'est un dispositif de ce type que concerne la présente invention.

L'état de l'art dans ce domaine fait apparaître plusieurs types de mécanismes :
- le plus classique est celui d'une barre venant en contact avec un pion central vertical de forme variable ;
- d'autres mécanismes font appel à une came coopérant avec une butée centrale qui présente un profil complémentaire de cette came ; par exemple, le troisième condyle fémoral central situé entre les deux condyles latéraux ;
- dans d'autres configurations prothétiques, il peut s'agir d'un troisième condyle central, lui-même en forme de cylindre transversal engagé dans un cylindre transversal se terminant à sa partie antérieure en forme de came.

Cette dernière configuration a pour effet néfaste une absence totale de liberté de mouvement antéro-postérieur entre la pièce fémorale et l'insert tibial, lorsque le cylindre possède le même axe transversal que les deux condyles latéraux. Il s'agit ainsi d'un mécanisme dit de type charnière, pouvant être responsable de pressions élevées au niveau de l'articulation entre la rotule et le fémur. Le mécanisme de roulement-glissement n'est pas respecté et l'une des conséquences peut être l'existence de douleurs rotuliennes importantes. Le rattrapage d'une certaine mobilité avant/arrière dans la zone de contact entre insert en polyéthylène et plateau tibial n'est pas suffisant pour palier cet inconvénient ; cette mobilité située à un niveau différent du niveau normal génère un mouvement parasite dit du "roll-forward", au cours duquel le tibia recule en flexion par rapport au fémur, ce qui peut entraîner le déboîtement des pièces prothétiques.

Dans une configuration de ce type de prothèse (voir WO 98/46171 A), la saillie médiane comporte à sa face supérieure une courbure guidante, prenant appui sur une surface complémentaire située entre les deux condyles fémoraux produisant un effet de came, lequel développe une déplacement optimal du fémur par rapport au tibia. Cependant, l'emboîtement de ce dispositif dans le plan frontal reproduit une courbe brisée, source de chocs, de contact angulaire ("edge-contact"), d'arrêt dur et donc de mécanismes pouvant entraîner le descellement de la prothèse.

La présente invention est donc basée sur une prothèse à plateau mobile, le plateau ou insert étant totalement congruent dans le sens transversal par sa surface supérieure avec les surfaces articulaires du fémur et par sa face inférieure avec la face supérieure de l'embase métallique implantée dans le tibia.

Parmi les prothèses avec plateau mobile congruent existant actuellement, la plupart possèdent une congruence dans les zones proches de l'extension, mais cette congruence diminue de façon importante lors de la flexion à cause de la diminution progressive des rayons de courbure condyliens, dans la partie postérieure de l'articulation, alors que le rayon de courbure du plateau tibial reste constant. Ceci est dû au rayon polycentrique du condyle dans le plan sagittal.

D'autres implants possèdent des surfaces de contact élevées de l'extension à la flexion. Cependant, dans ces réalisations, les différents plans sagittaux (dans le plan XY) sont décalés les uns par rapport aux autres, ce qui, en vue de face, se traduit par une ligne brisée, susceptible de générer des pics de contrainte élevés au niveau du polyéthylène, ainsi que des transmissions de contraintes anormalement élevées, de chocs, et de vibrations aux sites de fixation de la prothèse lors des mouvements de roulis / inclinaison latérale.

En général, l'appui des prothèses de genou se fait par le contact entre les condyles fémoraux et les glènes tibiales, avec un appui médial et un appui latéral. Du fait que l'axe mécanique du corps allant du centre de gravité du corps vers le contact du pied au sol passe de façon médiale par rapport à l'articulation du genou, les appuis sur les deux compartiments médial et latéral du genou sont asymétriques, créant plusieurs sources de problèmes :

L'un d'eux réside dans le fait qu'un couple de descellement est créé par une compression dans le compartiment interne nettement supérieure à la compression dans le compartiment externe. De ce fait, le compartiment externe du genou a tendance à se relever au niveau de sa fixation tibiale et à entraîner une possibilité de décollement de cette dernière, source de mobilisation de la prothèse, pouvant conduire à une détérioration et à un nouvel acte chirurgical.

Une autre source de problèmes réside dans le fait qu'il existe entre la phase d'appui et la phase oscillante du pas un décollement de la pièce métallique fémorale par rapport à l'insert en polyéthylène, que l'on appelle en anglais "lift-off" et que l'on peut traduire en français par effet de "roulis". Ce "lift-off" se manifeste principalement entre 40 et 70° de flexion du genou, principalement dans la zone de 50 à 70°. Il s'agit en général du décollement du condyle latéral par rapport au plateau tibial latéral. Ce "lift-off", qui existe de façon physiologique (environ 1,8 mm), peut atteindre des amplitudes de plusieurs millimètres, voire 5 mm ou plus, dans certains cas de prothèses.

Dans la forme d'appui élaborée selon l'invention, par le biais d'emboîtements concaves convexes dans les plans frontal et sagittal, l'appui se fait par les condyles latéraux et sur le dôme central ainsi que sur les versants latéraux obliques de ce dôme, ce qui, comme on le verra ci-après, est l'une des originalités principales de l'invention.

Dans le concept proposé selon l'invention, la résultante des transmissions des contraintes se fait préférentiellement à la partie centrale de l'embase tibiale, autour de son élément central de fixation que l'on appelle quille plantée au centre de l'extrémité supérieure de l'os tibial. Ce type de transmission a pour but de réduire au maximum l'effet de couple médio latéral responsable du descellement ou bien d'une usure prédominant sur l'un des deux compartiments.

L'emboîtement concave convexe dans les plans frontal et sagittal entre la dépression centrale du fémur et le tore (la saillie médiane de l'insert) a pour bénéfice d'offrir un appui congruent et progressif au cours des mouvements de lift-off et ce quel que soit l'angle de flexion du genou.

Cette forme de contact entre la pièce métallique fémorale et l'insert en polyéthylène permet en outre de faire varier les contacts de façon continue par l'engagement progressif de la surface de contact en évitant les chocs et les vibrations qui, lorsqu'ils sont transmis aux ancrages des pièces métalliques dans l'os tibial ou fémoral, sont source de vibrations favorisant là encore le descellement des pièces prothétiques qui conduit au changement de cette dernière.

La présente invention vise à proposer une prothèse du genou qui ne présente pas les inconvénients rappelés ci-dessus des prothèses de la technique antérieure.

La prothèse selon l'invention vise deux objectifs :
- la congruence destinée à protéger la longévité du polyéthylène utilisé et à en diminuer l'usure ;
- une cinématique destinée à donner un fonctionnement confortable pour le patient.

### LA CONGRUENCE

- une congruence avec une grande surface de contact entre le composant fémoral et l'insert, quel que soit l'angle de flexion, permet de diminuer les pressions exercées sur le polyéthylène ;
- la congruence doit diminuer au cours de la flexion, la grande surface de contact étant nécessaire dans le secteur d'appui de 0 à 60°, mais le secteur de flexion au-delà de 90° ne nécessite pas une grande surface de contact car :
   - l'utilisation de ce secteur est plus rare ;
   - la congruence étendue à tous les degrés de flexion, en particulier au-delà de 90° à pour conséquence une prothèse très contrainte, donc un risque de sollicitation excessive des ancrages ;
- la congruence de surface élevée est nécessaire, mais sans contact angulaire et sans zone de méplat ou de rebroussement du type tenon et mortaise intervenant lors des mouvements de LIFT-OFF ou d'inclinaison latérale, ce quelque soit l'angle de flexion : C'est la congruence frontale.

### LA CINEMATIQUE

- la cinématique doit favoriser le bras de levier de l'appareil extenseur (efficacité du travail du muscle quadriceps par l'intermédiaire de la rotule et du tendon rotulien), afin de permettre une force efficace lors de la montée et de la descente des escaliers. Ceci est obtenu par un déport antérieur de l'appui rotulien sur la trochlée en extension.
- la cinématique doit respecter le roulement glissement vrai du genou. Celui-ci est défini de la façon suivante : le point de contact du composant fémoral par rapport à l'insert est de quelques millimètres en avant du milieu de cet insert en extension à 0° et recule de quelques mm en arrière du milieu de l'insert lorsque la flexion dépasse 15 à 20°, sans que le composant fémoral et le segment osseux fémoral ne se déplacent vers l'arrière par rapport au composant tibial ou au segment osseux tibial. Ceci se distingue du faux roulement glissement dans lequel le composant fémoral et l'os fémoral reculent par un effet de came par rapport au composant tibial de la prothèse ou par rapport à l'os tibial, phénomène que l'on doit éviter, car il est générateur :
   1°) de pressions anormalement élevées sur le polyéthylène de la rotule et de l'insert, sources d'usure précoce et de douleurs ;
   2°) de mouvements de translation d'avant en arrière du fémur sur l'insert, générateur de délamination sous la surface du polyéthylène et donc d'usure précoce de ce dernier.

Afin de respecter ces objectifs, la présente invention propose une nouvelle géométrie des surfaces de la pièce fémorale et de l'insert. Plus précisément l'invention concerne une prothèse de l'articulation du genou qui comporte :
- un système avec trois zones d'appui entre la pièce fémorale et l'insert ;
- un système ayant une continuité du contact entre les surfaces d'appui de la pièce fémorale et de l'insert en médio-latéral ;
- une succession de segments de surfaces concaves-convexes ;
- dans le plan frontal, une succession d'emboitements pièce fémorale- insert concave-convexe, puis convexe-concave, puis concave-convexe en allant de la partie médiale vers la partie latérale ;
- dans le plan sagittal, les trois surfaces fémorales - médiale, centrale et latérale - ont une convexité tournée vers le bas, alors que les trois surfaces de l'insert ont une concavité tournée vers le haut, de manière à avoir une zone centrale en forme de selle mais un contact médio-latéral continu.

L'invention a également pour but de proposer une prothèse de genou dans laquelle la forme générale des trois zones latérale, centrale et médiale de la pièce fémorale est déterminée par une courbe spirale dans le plan sagittal, la forme générale des trois zones latérale, centrale et médiale de l'insert étant également déterminées par une courbe spirale dans le plan sagittal, la courbe spirale génératrice de l'insert étant dérivée de la courbe spirale génératrice de la pièce fémorale. Ces deux courbes spirales sont calculées dans le but de reproduire le roulement glissement vrai selon la définition ci-dessus.

L'invention a également pour but une prothèse de genou dans laquelle la congruence des surfaces articulaires entre la pièce fémorale et l'insert dans le plan frontal est assurée par une succession de surfaces courbes continues au niveau des trois zones d'appui, surfaces courbes raccordées entre elles sans aucune discontinuité ni arrête vive ou ligne de rebroussement ni méplat, permettant ainsi un déplacement en roulis en translation et en inclinaison dans le plan frontal entre la pièce fémorale et l'insert, au cours duquel la surface de contact sera toujours congruente quel que soit l'angle de flexion. Au sens de la présente invention, on considère que la surface de la prothèse comporte un méplat si, en un point de celle-ci, la surface de la prothèse suit, suivant plus d'un millimètre, au moins trois tangentes à cette surface, définissant un plan.

L'invention a enfin pour but une prothèse de genou dans laquelle les appuis sont transmis de façon continue de la partie médiale aux parties latérales de la prothèse au cours du cycle de la marche, avec une mise en charge sans à-coup, sans risques de vibration ou de transmission brutale des contraintes en apportant au patient un effet de souplesse confortable et en lui évitant de percevoir des chocs.

A cet effet, l'invention a pour objet une prothèse du genou du type comportant une pièce fémorale, généralement métallique, apte à être implantée dans le fémur, une pièce tibiale, généralement métallique, apte à être implantée dans le tibia, et une pièce intermédiaire ou insert, en une matière plastique telle que le polyéthylène, interposée entre la pièce tibiale et la pièce fémorale, l'insert pouvant être rigidement solidaire de la pièce tibiale ou mobile en rotation autour d'un axe vertical par rapport à celle-ci, la pièce fémorale comportant, d'une part, deux parties latérales, aptes à prendre appui et à se déplacer dans deux cavités latérales de profil approprié de l'insert, et, d'autre part, une partie centrale en creux disposée entre les parties latérales et apte à prendre appui sur une partie centrale en saillie de l'insert, la partie centrale en saillie de la surface de l'insert tournée vers la pièce fémorale, ayant une forme convexe, vue de face, et une forme concave, vue de profil depuis l'avant jusqu'à l'arrière de l'insert, tandis que la partie centrale de la pièce fémorale a une forme concave, vue de face, et convexe, vue de profil, depuis l'avant jusqu'à l'arrière de celle-ci, lui permettant de chevaucher la partie en saillie de l'insert au cours de ses déplacements relatifs et de coopérer avec elle à la manière d'une came, cette prothèse étant caractérisée en ce que :
- les surfaces de la pièce fémorale et de l'insert appelées à entrer en contact au cours des mouvements relatifs des deux pièces ne comportent aucune discontinuité, ni arête, et coopèrent par chevauchement de parties concaves et de parties convexes au cours de la totalité de leurs mouvements dans chacun des plans sagittal et frontal ;
- en coupe dans un plan frontal, la surface de l'insert tournée vers la surface correspondante de la pièce fémorale est une courbe qui comporte une portion centrale ondulée en forme de selle, dont la convexité est tournée vers la pièce fémorale et qui se raccorde de chaque côté à une partie en creux, ayant une forme correspondant sensiblement à celle de la partie associée de la pièce fémorale, l'ensemble formant une courbe ondulée sans discontinuité, ni arête, du type général d'une sinusoïde, tandis que, en coupe dans un plan frontal, la surface de la partie fémorale tournée vers la surface correspondante de l'insert et entrant en contact continu médio-latéral avec cette dernière est une courbe, qui comporte une portion centrale ondulée, dont la concavité est tournée vers l'insert et qui se raccorde tangentiellement de chaque côté aux parties latérales de la pièce fémorale, l'ensemble formant une courbe ondulée sans discontinuité, ni arête vive, du type général d'une sinusoïde.

Dans cette prothèse, laq surface fémorale est développée à partir d'une courbe spirale dans le plan sagittal parcourant une courbe ondulée de type sinusoïdal dans le plan frontal et la surface de l'insert est développée à partir d'une courbe spirale dans le plan sagittal parcourant une courbe de type sinusoïdal dans le plan frontal, les deux surfaces ayant un emboîtement concave-convexe dans chacun des deux plans.

Les surfaces de la pièce fémorale et de l'insert appelées à entrer en contact au cours des mouvements relatifs des deux pièces sont donc des portions de surface qui ne comportent aucune discontinuité, ni arête, ni méplat, ni zone de rebroussement, et qui coopèrent par chevauchement de parties concaves et de parties convexes au cours de la totalité des mouvements dans chacun des plans sagittal et frontal, quel que soit l'angle de flexion.

Les surfaces de la pièce fémorale et de l'insert disposées en regard sont destinés à autoriser les mouvements :
- dans un plan frontal : mouvement dit de"lift off', c'est-à-dire de soulèvement glissant et d'angulation de l'une des parties latérales de la pièce fémorale, avec un contact restant congruent entre la pièce fémorale et l'insert, tant dans la cavité latérale de l'insert que sur tout ou partie de la saillie centrale de ce dernier, ce quel que soit l'angle de flexion ;
- dans un plan sagittal : un mouvement de flexion avec préférentiellement un roulement glissement vrai de la pièce fémorale sur l'insert, c'est-à-dire un déplacement du point de contact de la pièce fémorale sur l'insert depuis quelques millimètres en avant du centre de l'insert, en position d'extension 0, jusqu'à quelques millimètres en arrière du centre de l'insert, en position fléchie, mais sans déplacement de la pièce fémorale elle-même ou du segment osseux qui la porte par rapport a la pièce tibiale (absence de translation) ;
- dans un plan horizontal : rotation par rapport à un axe vertical dont l'amplitude varie suivant que l'insert est mobile en rotation ou non par rapport à la pièce tibiale ; selon l'invention, l'insert est de préférence libre en rotation axiale.

Plus précisément, vue en coupe par le plan frontal, la surface de l'insert tournée vers la surface correspondante de la pièce fémorale et entrant en contact continu dans le sens médiolatéral (transversal) avec cette dernière comporte deux segments de courbe latéraux à concavité tournée vers le haut et un segment de courbe central dont la convexité est tournée vers la pièce fémorale, la convexité de la partie centrale se raccordant de chaque côté aux parties concaves avec un profil qui correspond sensiblement à la partie latérale associée de la pièce fémorale, l'ensemble formant une courbe sans discontinuité ni arrête, ni méplat, apparentée à une sinusoïde, depuis l'avant jusqu'à l'arrière dudit insert.

De même, vue en coupe dans un plan frontal, la pièce fémorale a un profil complémentaire depuis l'avant jusqu'à l'arrière de ses parties latérales. Autrement dit, il existe une courbe médiale convexe tournée vers le bas, puis une concavité centrale chevauchant la partie centrale de l'insert, puis une convexité latérale tournée vers le bas, ces trois segments de courbe entrant en contact continu dans le sens médiolatéral (transversal) avec les surfaces correspondantes de l'insert.

Une prothèse de l'art antérieur (voir brevet US N° 4 470 158) comporte également une pièce fémorale déterminée par deux courbes génératrices, une courbe frontale parcourant une courbe sagittale. Cependant, cette prothèse antérieure se différencie fondamentalement de celle de la demande en ce que la courbe génératrice frontale est un élément géométrique du dessin mais ne correspond pas totalement à la zone de contact entre les pièces prothétiques (fémur et insert). En effet, la pièce fémorale de cette prothèse ne comporte que deux condyles classiques, séparés par une zone discontinue (échancrure), alors que, selon la présente invention, il existe une continuité totale d'un bord à l'autre de la pièce fémorale.

Dans ce brevet U.S., la portion de courbe génératrice appelée K3 est un élément de dessin, mais pas une zone de matière continue et d'appui de la pièce fémorale sur l'insert, comme dans la prothèse selon l'invention. La courbe génératrice frontale de la prothèse U.S. parcourt une courbe polycentrique sagittale, qui comporte quatre segments de rayons différents d'avant en arrière, alors que, selon la présente invention, la courbe frontale parcourt une courbe génératrice spirale.

Dans une autre prothèse de la technique antérieure (brevet US N° 5 609 639 de Walker), une pièce fémorale présente une continuité dans la partie située entre les deux « condyles» et entre en contact avec un composant "méniscal" ayant une portion centrale incurvée, dont la convexité est tournée vers la pièce fémorale et se raccorde avec deux parties latérales en creux. Il est cependant indiqué que le composant fémoral a un rayon constant dans le plan sagittal et que le composant "méniscal" a soit un rayon sagittal constant, la mobilité d'avant en arrière se faisant entre le composant "méniscal" et le plan tibial, soit un rayon sagittal variable, déterminé par l'empreinte des mouvements d'avant en arrière creusée par le composant fémoral à rayon sagittal constant. Ceci ne peut générer ni les formes, ni aucune des fonctions de mouvement ou de contact permis par l'emboîtement d'une spirale dans une spirale.

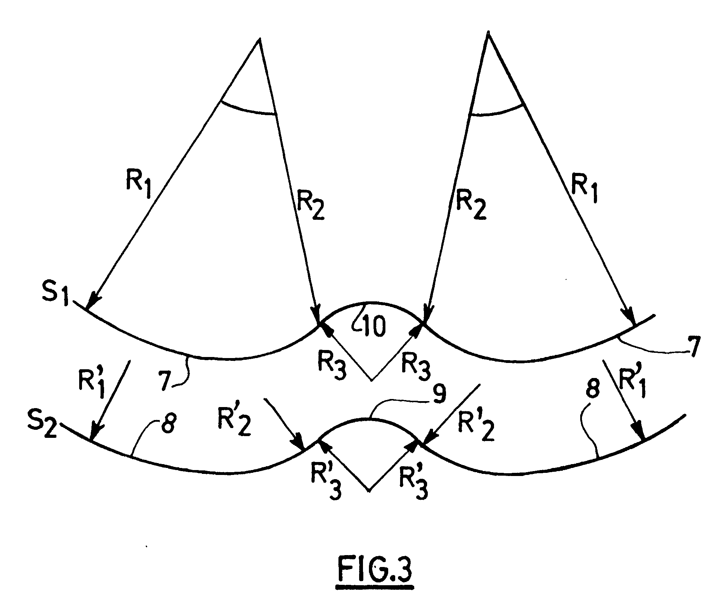

Dans le plan frontal, le composant fémoral du brevet Walker a une forme "condylienne", donc anatomique, et une conformité avec les cavités latérales du composant méniscal, donc également anatomiques, ce qui le distingue de la présente invention. Dans ce brevet, il n'est donné aucune description géométrique des contacts, dans la partie centrale, entre le composant fémoral et le composant "méniscal", autrement que par une continuité du contact. Si l'on se réfère aux figures 3(b) et 3(c) de ce brevet, on constate qu'il existe à la partie postérieure de l'insert une zone centrale aplatie se raccordant par des bords angulés avec les parties latérales, ce qui est incompatible avec les propriétés des surfaces du composant fémoral et de l'insert selon la présente invention, ainsi qu'avec les avantages cinématiques et tribologiques qui en découlent. De même, la trochlée du composant fémoral de la figure 3(b) est aplatie et ne comporte pas de forme concave convexe à sa partie centrale. Elle doit donc se raccorder avec des angulations aux autres surfaces .

Enfin, toujours selon la technique antérieure, le brevet FR N° 2 621 1 243 décrit une prothèse dont le composant tibial a une forme de selle de cheval en sa partie centrale, le composant fémoral n'entrant en contact que par un contact ponctuel avec le composant tibial, et uniquement sur la partie centrale, sans qu'il y ait de contact entre les parties latérales du fémur et l'élément tibial. Par ailleurs, la forme de la selle tibiale dans le plan sagittal est simplement convexe et n'est pas définie par une courbe de type spirale. Elle ne possède donc pas les avantages liés à l'emboîtement d'une spirale dans une spirale lors de la flexion du genou selon un axe transversal.

Dans la pratique, dans la prothèse selon la présente invention, la forme précise à adopter pour les courbures spirales de l'insert et de la surface centrale de la pièce fémorale dans le plan sagittal est définie à partir de radiographies du genou du patient à diverses positions de flexion, de manière à reproduire aussi exactement que possible le déplacement du point de contact de l'articulation naturelle en roulement glissement vrai, c'est-à-dire un déplacement du point de contact sans déplacement des segments osseux ou des composants prothétiques.

De même, la forme précise à adopter pour la courbe dans le plan frontal est définie par la nécessité d'une angulation de 5° environ entre la pièce fémorale et l'insert, lors du soulèvement latéral dit "lift-off" (ou varus - valgus).

Ceci distingue la prothèse selon l'invention de celle de WO 98/46171 A, dans laquelle, bien qu'il existe une continuité avec trois surfaces d'appui - deux « condyles » et une came « intercondylienne » - la conformité dans le plan frontal ne concerne que les cavités latérales de l'insert et les « condyles » correspondants, mais pas la partie centrale.

Les dessins schématiques annexés illustrent de façon plus détaillée une forme de réalisation de l'invention. Ils n'ont naturellement aucun caractère limitatif. Sur ces dessins :
La figure 1 est une vue en perspective éclatée d'une prothèse du genou conforme à l'invention ;
La figure 2 est une vue schématique à plus grande échelle illustrant la forme sensiblement complémentaire des surfaces de contact en regard de l'insert et de la pièce fémorale ; et
La figure 3 représente des coupes par un plan frontal des surfaces de contact en regard de l'insert et de la pièce fémorale.

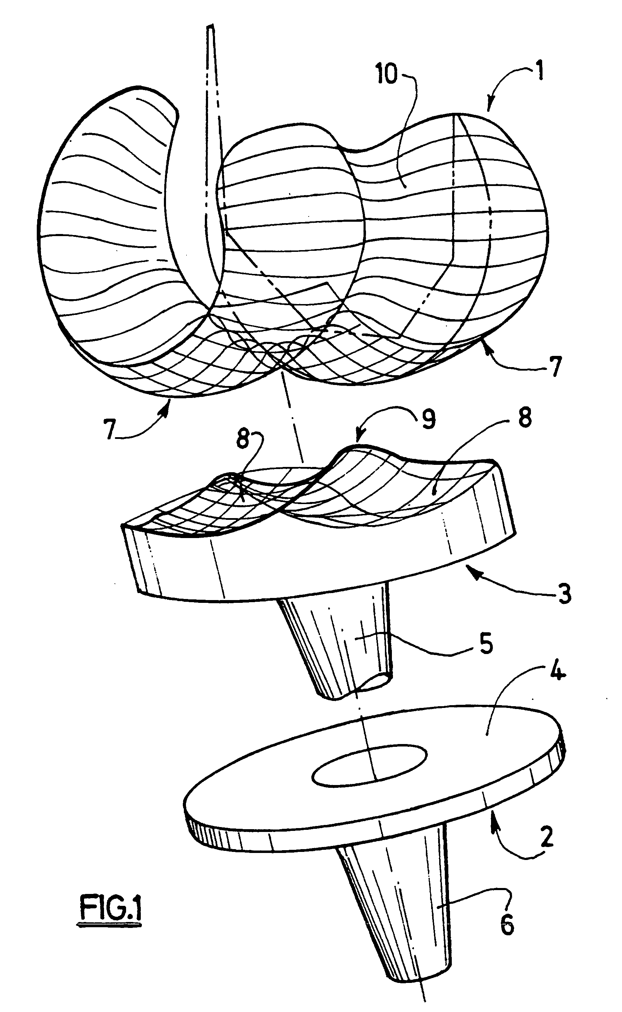

La prothèse du genou illustrée par la figure 1 comprend une pièce fémorale 1, généralement métallique, apte à être implantée dans le fémur du patient, une pièce tibiale 2, également métallique, apte à être implantée dans le tibia du patient, et un insert 3, généralement en une matière plastique telle que le polyéthylène.

L'insert 3 prend appui sur un plateau 4 de la pièce tibiale 2 et peut être fixé en position sur celle-ci ou, comme représenté sur le dessin, mobile en rotation par rapport au plateau 4 autour d'un axe qui, en position d'utilisation de la prothèse, est disposé verticalement. Dans ce but, l'insert 3 comporte ici, faisant saillie à partir de sa surface tournée vers la pièce 2, un pion 5 engagé dans une quille centrale creuse 6 de la pièce tibiale 2, de façon connue en soi.

De façon usuelle, la pièce fémorale 1 comporte deux parties latérales 7 dont la section, en coupe par un plan sagittal, a la forme d'une spire, dont l'équation mathématique exacte est étrangère à la présente invention.

La pièce fémorale 1 est destinée à se déplacer préférentiellement suivant un mouvement de roulement-glissement des parties latérales 7 sur les parties en creux 8 de la face de l'insert 3 tournée vers la pièce 1, qui ont des formes sensiblement correspondantes, également à profil de spire vues en coupe dans un plan sagittal.

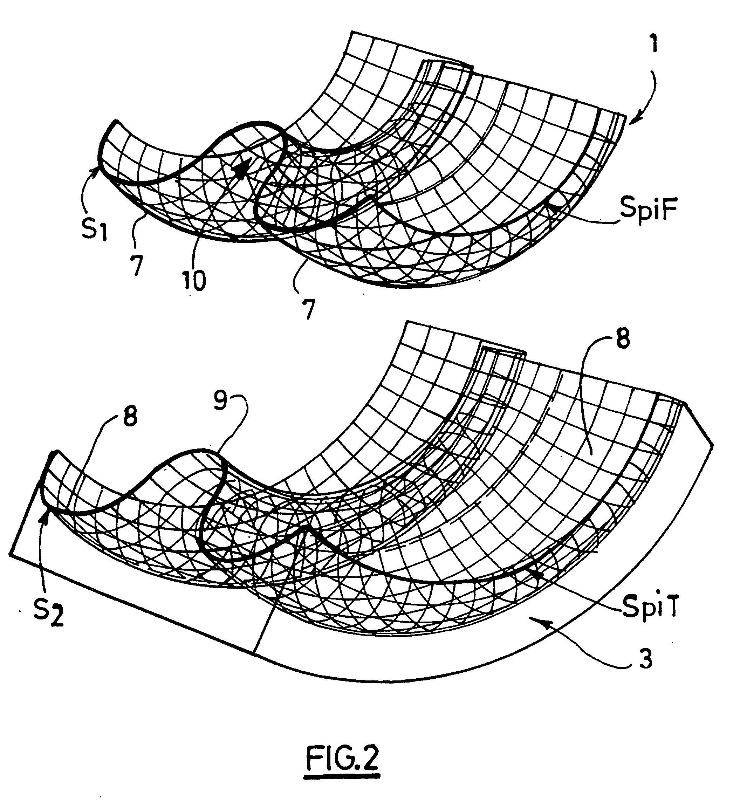

Conformément à l'invention, l'insert 3 présente dans sa partie centrale une partie dorsale en saillie 9, formant came, qui, vue de face (voir figures 2 et 3), présente un profil ondulé dont la convexité est tournée vers la pièce fémorale, tandis que, en vue de côté, elle présente un profil concave en forme de selle, cette partie 9 en saillie ne comportant aucune discontinuité, angulation ou arête, et se raccordant tangentiellement dans toutes les directions aux surfaces 8 contiguës, également sans discontinuité, angulation ou arête. La partie 10 de la pièce fémorale 1 a une forme sensiblement complémentaire de celle de la partie 9 de l'insert qu'elle chevauche de l'extension à la flexion complète et, elle aussi, se raccorde tangentiellement aux parties latérales 7 dans toutes les directions, sans discontinuité, ni angulation ni méplat ni arête.

Ce chevauchement de la pièce fémorale sur l'insert 3 se traduit donc par un emboîtement concave-convexe des deux pièces dans un plan sagittal et un emboîtement concave-convexe des deux pièces dans un plan frontal, autorisant cependant une translation antéro-postérieure de la pièce fémorale 1 par rapport à l'insert 3, le guidage se faisant par l'engagement de la partie 9 en saillie de l'insert, dont la surface supérieure en coupe dans un plan sagittal est concave, dans la voûte intercondylienne 10 de la pièce fémorale 1, et dont la face inférieure médiane est convexe dans un plan sagittal ce processus ayant lieu tout au long du mouvement de flexion extension.

Cet emboîtement concave-convexe médian permet également l'arrêt des déplacements anormaux (subluxation ou luxation) vers l'avant ou vers l'arrière du fémur par rapport au tibia et vice-versa, avec l'avantage d'une progressivité de cet arrêt, du fait de la continuité des surfaces en contact mutuel.

La forme précise de la partie en saillie 9 formant came dans le plan sagittal est déterminée en procédant à des radiographies de l'articulation du genou, replié en un grand nombre de positions différentes, et elle n'est pas définie par une équation mathématique précise. Cette came est telle qu'à un angle de flexion de 0°, le centre des appuis de la pièce fémorale sur l'insert soit de quelques millimètres en avant du centre de l'insert et qu'il soit de quelques millimètres en arrière de ce centre, à partir d'un certain degré de flexion compris entre 15° et 20°.

Les différentes surfaces, respectivement de la pièce fémorale et de l'insert, sont simplement tangentes entre elles dans tous les plans, sans aucune zone de rupture, la surface de contact se déplaçant de l'avant vers l'arrière de l'insert, comme le veut la physiologie.

La figure 2 montre les surfaces générées par les deux courbes S1 et Spi F, pour la surface de contact de la pièce fémorale, et S2 et Spi T, pour la surface de contact de l'insert.

Comme indiqué, ci-dessus les courbes Spi F et Spi T ont une forme de spirale, sans que ce terme implique une définition mathématique précise. De même en coupe dans un plan frontal, la surface de contact S1 de la pièce fémorale 1 et la surface de contact S2 de l'insert (voir figure 3) ont un profil sinusoïdal, sans que ce terme implique une équation mathématique précise.

On notera que la partie centrale en creux 10 de la pièce fémorale 1 peut se raccorder par une courbure de rayon R₂ constant d'avant en arrière avec les parties latérales 7 de cette pièce 1. De même, la partie centrale convexe 9 de l'insert 3 tournée vers la pièce fémorale 1 peut se raccorder par une courbure de rayon R'₂ constant d'avant en arrière avec les parties latérales en creux 8 de cet insert.

La prothèse conforme à l'invention présente l'avantage de respecter la cinématique physiologique du genou, c'est-à-dire le mouvement préférentiel de roulement-glissement de l'articulation, avec recul du point de contact de la pièce fémorale sur l'insert, de l'extension vers la flexion, ce qui optimise le moment d'action de l'appareil extenseur et donc la force de propulsion du genou, lors d'une montée, d'une descente et d'un relèvement.

Elle présente également l'avantage :
- de garder une surface de contact élevée au cours de la flexion du genou, ce qui génère comme corollaire une pression basse dans le polyéthylène et donc une usure faible de la prothèse ;
- de conserver une congruence lors des mouvements d'inclinaison dans le plan frontal, donc une absence de chocs et de mécanismes d'arrachement ou de descellement ;
- de conférer à ce dispositif une bonne stabilité des pièces l'une par rapport à l'autre, lors des déplacements d'avant en arrière et d'inclinaison latérale.
- De garder une surface de contact totale médio-latérale de l'extension à la flexion complète, surface diminuant progressivement par la mise en contact de courbes spirales.

## Revendications

1. Prothèse du genou du type comportant une pièce fémorale (1), généralement métallique, apte à être implantée dans un fémur, une pièce tibiale (2), généralement métallique, apte à être implantée dans un tibia, et un insert (3), en une matière plastique telle que le polyéthylène, interposée entre la pièce tibiale (2) et la pièce fémorale (1),
- l'insert (3) pouvant être rigidement solidaire de la pièce tibiale (2) ou mobile en rotation autour d'un axe vertical par rapport à celle-ci, et comportant une surface de contact tournée vers la pièce fémorale (1), la surface de contact de l'insert (3) formant, en coupe dans un plan sagittal, une concavité depuis l'avant de l'insert (3), correspondant à la face avant du (SpiT) genou, jusqu'à l'arrière de l'insert (3), correspondant à la face arrière du genou, et en coupe dans un plan frontal, une courbe (S2) comportant une partie centrale en saillie (9), ayant une convexité tournée vers la pièce fémorale (1), cette partie centrale en saillie (9) se raccordant sur chacun de ses côtés à une partie en creux (8), pour former une courbe ondulée, et
- la pièce fémorale (1) comportant une surface de contact comprenant, d'une part, deux parties latérales (7), aptes à prendre appui et à se déplacer dans deux cavités latérales (8) de profil approprié de l'insert (3), et, d'autre part, une partie centrale en creux (10) disposée entre les parties latérales (7) et apte à prendre appui sur la partie centrale en saillie (9) de l'insert (3), la surface de contact de la pièce fémorale (1) formant, en coupe dans un plan sagittal, une convexité depuis l'avant jusqu'à l'arrière, et en coupe dans un plan frontal, une courbe (S1) comportant une portion correspondant à la partie centrale en creux (10), cette portion ayant une concavité tournée vers l'insert (3) et se raccordant sur chacun de ses côtés aux portions correspondant aux deux parties latérales (7),
**caractérisée en ce que** :
- dans un plan frontal, lesdites courbes (S1 et S2) décrivent une succession d'emboîtement concave-convexe, puis convexe-concave et concave-convexe, en allant de l'une vers l'autre des parties latérales (7) depuis l'avant jusqu'à l'arrière de la pièce fémorale (1) et de l'insert (3), et **en ce que**
- les surfaces de contact de la pièce fémorale (1) et de l'insert (3) ne comportent à l'intérieur de ces surfaces aucune discontinuité, ni arête, ni angulation, ni méplat et coopèrent par contact continu de chacune des deux parties latérales (7) et de la partie centrale en creux (10) de la pièce fémorale (1), avec chacune des parties correspondantes de l'insert (3), au cours de la totalité de leurs mouvements dans chacun des plans sagittal et frontal.

2. Prothèse selon la revendication 1, **caractérisée en ce que** :
- la surface de contact de la pièce fémorale (1) est développée, depuis l'avant jusqu'à l'arrière de la pièce fémorale (1), à partir d'une courbe spirale dans le plan sagittal, parcourant dans le plan frontal la courbe (S1) comportant ladite portion correspondant à la partie centrale en creux (10), et
- la surface de contact de l'insert.(3) est développée, depuis l'avant jusqu'à l'arrière de l'insert (3), à partir d'une courbe spirale dans le plan sagittal, parcourant dans le plan frontal la courbe (S2) comportant ladite portion correspondant à la partie centrale en saillie (9).

3. Prothèse selon l'une des revendications 1 et 2, **caractérisée en ce que** la partie centrale convexe (9) de l'insert (3) se raccorde par une courbure de rayon (R'₂) constant d'avant en arrière avec les deux cavités latérales (8) de cet insert (3).

4. Prothèse selon l'une des revendications précédentes, **caractérisée en ce que** la partie centrale en creux (10) de la pièce fémorale (1) se raccorde par une courbure de rayon (R₂) constant d'avant en arrière, avec les parties latérales (7).

5. Prothèse selon l'une des revendications précédentes, **caractérisée en ce que** la partie en saillie (9) de l'insert (3) et la partie en creux (10) de la pièce fémorale (1) ont, dans le plan sagittal, deux courbures qui coopèrent à la manière d'une came et qui sont déterminées à partir de radiographies du mouvement de l'articulation du genou, cette came étant telle qu'à un angle de flexion de 0°, le centre des appuis de la pièce fémorale (1) sur l'insert (3) soit de quelques millimètres en avant du centre de cet insert (3) et qu'il recule de quelques millimètres en arrière du centre de l'insert, à partir d'un certain degré de flexion compris entre 15° et 20°.

## Patentansprüche

1. Knieprothese des Typs, der ein im allgemeinen metallisches Femoralteil (1), das zum Implantieren in einen Oberschenkelknochen geeignet ist, ein im allgemeinen metallisches Tibialteil (2), das zum Implantieren in ein Schienbein geeignet ist, und einen Einsatz (3) aus einem Kunststoffmaterial wie Polyethylen enthält, der zwischen dem Femoralteil (1) und dem Tibialteil (2) eingesetzt ist,
wobei der Einsatz (3) fest mit dem Tibialteil (2) verbunden oder drehbar um eine senkrechte Achse relativ zu diesem beweglich sein kann und eine dem Femoralteil (1) zugewandte Kontaktfläche aufweist,
wobei die Kontaktfläche des Einsatzes (3) geschnitten in einer Sagittalebene eine Konkavität bildet von der Vorderseite des Einsatzes (3) aus, die der Vorderseite des (SpiT) Knies entspricht, bis zu der Rückseite des Einsatzes (3), die der Rückseite des Knies entspricht, und geschnitten in einer Frontalebene eine Kurve (S2), die einen vorspringenden Mittelteil (9) enthält, der eine dem Femoralteil (1) zugewandte Konvexität aufweist, wobei dieser vorspringende Mittelteil (9) sich auf jeder seiner Seiten an einen Vertiefungsabschnitt (8) anschließt, um eine gewellte Kurve zu bilden, und
wobei das Femoralteil (1) eine Kontaktfläche aufweist, die einerseits zwei Seitenabschnitte (7) enthält, die dazu geeignet sind, in den zwei seitlichen Vertiefungen (8) des entsprechenden Profils des Einsatzes (3) Auflage zu nehmen und sich zu bewegen, und andererseits einen vertieften Mittelabschnitt (10), der zwischen den Seitenabschnitten (7) angeordnet ist und dazu geeignet ist, auf dem vorspringenden Mittelteil (9) des Einsatzes (3) Auflage zu nehmen,
wobei die Kontaktfläche des Femoralteils (1) geschnitten in einer Sagittalebene eine Konvexität von der Vorderseite bis zu der Rückseite bildet und geschnitten in einer Frontalebene eine Kurve (S1), die einen Abschnitt enthält, der dem vertieften Mittelabschnitt (10) entspricht, wobei dieser Abschnitt eine dem Einsatz (3) zugewandte Konkavität aufweist und sich auf jeder seiner Seiten an Abschnitte anschließt, die den zwei Seitenabschnitten (7) entsprechen;
**dadurch gekennzeichnet, dass**
die Kurven (S1 und S2) in einer Frontalebene ausgehend von dem einen hin zu dem anderen Seitenabschnitt (7) von der Vorderseite bis zu der Rückseite des Femoralteils (1) und des Einsatzes (3) eine Aufeinanderfolge des Ineinandergreifen konkav-konvex, dann konvex-konkav und konkav-konvex beschreibt,
und dadurch, dass
die Kontaktflächen des Femoralteils (1) und des Einsatzes (3) im Inneren dieser Oberflächen weder irgendeine Diskontinuität noch eine Kante noch einen Winkel noch eine Abflachung aufweisen und zusammenwirken durch ständigen Kontakt jedes der zwei Seitenabschnitte (7) und des vertieften Mittelabschnitts (10) des Femoralteils (1) mit jedem der entsprechenden Abschnitte des Einsatzes (3) im Verlauf der Gesamtheit ihrer Bewegungen in jeder der Sagittal- und Frontal-Ebenen.

2. Prothese nach Anspruch 1, **dadurch gekennzeichnet, dass**
die Kontaktfläche des Femoralteils (1) von der Vorderseite bis zu der Rückseite des Femoralteils (1) gebildet wird ausgehend von einer Spiralkurve in der Sagittalebene, indem in der Frontalebene die Kurve (S1) durchlaufen wird, die den Abschnitt aufweist, der dem vertieften Mittelabschnitt (10) entspricht, und
die Kontaktfläche des Einsatzes (3) von der Vorderseite bis zu der Rückseite des Einsatzes (3) gebildet wird ausgehend von einer Spiralkurve in der Sagittalebene, indem in der Frontalebene die Kurve (S2) durchlaufen wird, die den Abschnitt aufweist, der dem vorspringenden Mittelabschnitt (9) entspricht.

3. Prothese nach Anspruch 1 oder 2, **dadurch gekennzeichnet, dass**
der konvexe Mittelabschnitt (9) des Einsatzes (3):sich über eine Krümmung mit konstantem Radius (R'₂) von vorne bis hinten an die zwei Vertiefungsabschnitte (8) des Einsatzes (3) anschließt.

4. Prothese nach einem der vorigen Ansprüche, **dadurch gekennzeichnet, dass**
der vertiefte Mittelabschnitt (10) des Femoralteils (1) sich über eine Krümmung mit konstantem Radius (R₂) von vorne bis hinten an die Seitenabschnitte (7) anschließt.

5. Prothese nach einem der vorigen Ansprüche, **dadurch gekennzeichnet, dass**
der vorspringende Mittelabschnitt (9) des Einsatzes (3) und der vertiefte Mittelabschnitt (10) des Femoralteils (1) in de Sagittalebene zwei Krümmungen aufweisen, die nach Art einer Nocke zusammenwirken und die aus Radiographien der Gelenkbewegung des Knies bestimmt werden,
wobei die Nocke so ist, dass sich das Auflagezentrum des Femoralteils (1) auf dem Einsatz (3) bei einem Beugungswinkel von 0° um einige Millimeter vor der Mitte des Einsatzes (3) befindet und dass es von einem bestimmten Beugungsgrad an, der zwischen 15° und 20° liegt, um einige Millimeter hinter die Mitte des Einsatzes (3) zurückweicht.

## Claims

1. A knee prosthesis of the type comprising a femoral component (1), generally of metal, capable of being implanted in a femur, a tibial component (2), generally of metal, capable of being implanted in a tibia, and an insert (3), of a plastics material such as polyethylene, interposed between the tibial component (2) and the femoral component (1),
- the insert (3) being capable of being rigidly integral with the tibial piece (2) or rotationally movable relative thereto around a vertical axis, and comprising a contact surface facing the femoral component (1), the contact surface of the insert (3) forming, in section in a sagittal plane, a concavity from the front of the insert (3), corresponding to the front face of the (Spi T) knee, to the rear of the insert (3), corresponding to the rear face of the knee and, in section in a frontal plane, a curve (S2) comprising a central projecting part (9), having a convexity facing the femoral component (1), said central projecting part (9) being connected on each of its sides with a recessed part (8), to form an undulating curve, and
- the femoral component (1) comprising a contact surface having, on the one hand, two lateral parts (7) capable of resting and moving in two lateral cavities (8) of suitable profile in the insert (3) and, on the other hand, a central recessed part (10) disposed between the lateral parts (7) and capable of resting on the central projecting part (9) of the insert (3), the contact surface of the femoral component (1) forming, in section in a sagittal plane, a convexity from the front to the rear and, in section in a frontal plane, a curve (S1) comprising a portioh corresponding to the central recessed part (10), this portion having a concavity facing the insert (3) and being connected on each of its sides to the portions corresponding to the two lateral parts (7),
**characterised in that**
- in a frontal plane, said curves (S1 and S2) describe a concave-convex, then convex-concave and concave-convex nesting sequence, passing from one to the other of the lateral parts (7) from the front to the rear of the femoral component (1) and the insert (3), and in' that
- the contact surfaces of the femoral component (1) and the insert (3) do not exhibit any discontinuity, or edge or angulation or flat part on the inside of said surfaces and cooperate by continuous contact of each of the two lateral parts (7) and of the central recessed part (10) of the femoral component (1) with each of the corresponding parts of the insert (3) throughout all their movements in each of the sagittal and frontal planes.

2. A prosthesis according to claim 1, **characterised in that**:
- the contact surface of the femoral component (1) is developed, from the front to the rear of the femoral component (1), on the basis of a spiral curve in the sagittal plane, passing through, in the frontal plane, the curve (S1) comprising said portion corresponding to the central recessed part (10), and
- the contact surface of the insert (3) is developed, from the front to the rear of the insert (3), on the basis of a spiral curve in the sagittal plane, passing through, in the frontal plane, the curve (S2) comprising said portion corresponding to the central projecting part (9).

3. A prosthesis according to either one of claims 1 and 2, **characterised in that** the convex central part (9) of the insert (3) is connected by a curvature of constant radius (R'₂) from front to rear with the two lateral cavities (8) of said insert (3).

4. A prosthesis according to any one of the preceding claims, **characterised in that** the central recessed part (10) of the femoral component (1) is connected by a curvature of constant radius (R₂) from front to rear with the lateral parts (7).

5. A prosthesis according to any one of the preceding claims, **characterised in that** the projecting part (9) of the insert (3) and the recessed part (10) of the femoral component (1) have, in the sagittal plane, two curvatures which cooperate in the manner of a cam and which are determined from radiographs of the movement of the knee joint, said cam being such that, at an angle of flexure of 0°, the centre of support of the femoral component (1) on the insert (3) is a few millimetres in front of the centre of said insert (3) and that it moves back a few millimetres behind the centre of the insert from a certain degree of flexure of between 15° and 20°.
